# EUROPEAN PATENT APPLICATION

(11) **EP 4 275 645 A1**
(43) Date of publication of application: **15.11.2023**
(21) Application number: 23167916.8
(22) Date of filing: 14.04.2023
(51) Int. Cl.: A61B 90/00, A61B 90/50, A61B 17/00

(54) **IMPROVED MULTIMEDIA DENTAL STATION**

(30) Priority: 15.04.2022 IT 202200007703
(71) Applicant: Riccardi, Luca, 61040 Monte Porzio (PU) (IT)
(72) Inventor: Riccardi, Luca, 61040 Monte Porzio (PU) (IT)
(74) Representative: Cutropia, Gianluigi

(57) **Abstract**

A multimedia dental station (100) comprises a camera (2) integrated into an operating lamp (1), a PC (5), and an OLED viewer (8) mounted in a prismatic helmet (7), wherein the OLED viewer (8) is connected to a receiver (70) to receive and display a first output signal (S4) coming from a signal switch (4) which received a screen duplication signal (S1) coming from the PC (5) and a second signal (S2) coming from a capture card (3) connected to the camera (2).

## Description

The present invention relates to a multimedia dental station.

Magnifying and/or illuminating systems are known in dentistry to facilitate the dentist in viewing the working field in the patient's oral cavity.

The most common magnifying systems are prismatic helmets or glasses with bi-ocular lenses that magnify the view of the oral cavity up to 5X. The helmet can be enlarged with a light source and/or with a miniature, USB-type camera with up to 8X digital zoom. The purpose of the helmet camera is only to store a digital file containing photos-audios/videos of the procedure performed by the dentist in an SD memory card. After the intervention, the digital file can be sent via e-mail, and the photos-audios/videos can be reproduced via a special reader and monitor.

Operating lamps with an integrated camera with digital or optical zoom that reproduces the live shooting of the dental intervention on a PC or/and on one or more monitors are known. Such operating lamps with an integrated camera make it possible to record and stream the dental intervention on the most popular video conferencing software platforms.

The videos and images related to the dental intervention can be stored and thus can be useful in assessing the ability and weaknesses of the operating team in order to continuously make appropriate adjustments to the operating technique used. For dentists, documenting their case history can be useful in order to communicate with colleagues or staff members during training meetings, such as conferences and cultural meetings. In addition, such archived documentation becomes extremely important in medical-legal litigations.

Another magnification system used by dentists is the operating microscope. The microscope work can be followed on a monitor in real time. However, there is no precise indication of how to use the microscope. Dentists are able to understand its usefulness based on their current practice.

Thus, the modern dental clinic is now moving toward a reality that certainly considers the magnified vision as the center of all dental activities, and not only of the endodontic practice.

US2018/249891 describes a medical system according to the preamble of claim 1. Such a system comprises a viewer mounted on a head-mounted display to display images taken by a camera; however, the system is not configured so that the viewer can display images taken by the camera enriched with other information from a PC.

US2017/273549 describes a portable surgical visualization kit comprising a camera to capture, viewing equipment configured to receive and display the captured images, and a processor to process the images.

US2021/306599 describes a dental and medical loupe system for lighting control, streaming and augmented reality assisted procedures.

The purpose of the present invention is to eliminate the drawbacks of the prior art by providing a multimedia dental station that is capable of lighting, shooting a procedure in the patient's oral cavity, storing such videos, and sharing such videos with the dentist, patient, and third parties (telemedicine).

Another purpose of the present invention is to provide such a multimedia dental station that is versatile, efficient, and user-friendly for the dentist and for some branches of medicine.

These purposes are achieved in accordance with the invention having the features listed in the appended independent claim 1.

Advantageous embodiments appear from the dependent claims.

Further features of the invention will become clearer from the following detailed description, which refers to merely illustrative and thus non-limiting embodiments, illustrated in the accompanying drawings, wherein:
Fig. 1 is a block diagram, illustrating a multimedia dental station according to the invention;
Fig. 2 is a perspective rendering view, illustrating a helmet assembly of the multimedia dental station according to the invention;
Fig. 3 is a block diagram, illustrating an OLED viewer, a transmission station and a power bank of the helmet assembly of Fig. 2;
Fig. 4 is a perspective view of the OLED viewer of the helmet assembly of Fig. 2;
Fig. 5 shows four transmitter modules of the OLED viewer, illustrating a front view and a PCB for each transmitter module;
Fig. 6 is a block diagram of the multimedia dental station according to the invention, illustrating some software programs installed in the PC of the multimedia dental station;
Fig. 7 is a schematic and perspective rendering view, illustrating a possible installation of the multimedia dental station of Fig. 1 with external devices;
Fig. 8 is a perspective rendering view, illustrating a first type of installation of the multimedia dental station according to the invention; and
Fig. 9 is a perspective rendering view, illustrating a second type of installation of the multimedia dental station according to the invention.

With the aid of the Figures, the multimedia dental station according to the invention is described, which is comprehensively indicated with reference numeral 100.

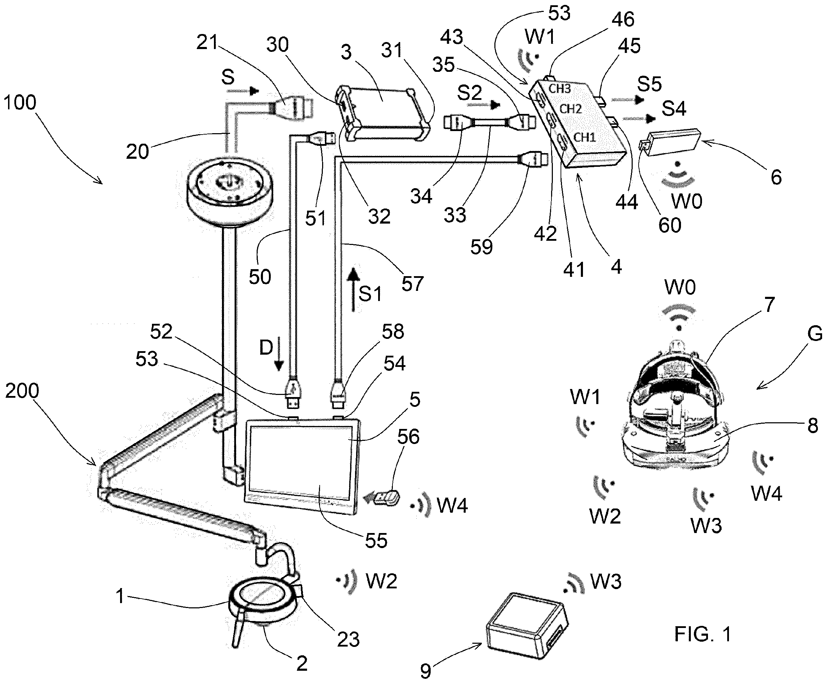

With reference to Fig. 1, the station (100) comprises an operating lamp (1), such as a surgical lamp.

A camera (2) is integrated into the operating lamp (1). The camera (2) is a digital video camera having an optical zoom of at least 30 X.

The operating lamp (1) is supported by an articulated system (200) in order to be moved and oriented by the dentist in appropriate positions to illuminate and shoot the patient's oral cavity.

The camera (2) is connected to a video capture card (3) with a cable (20).

The camera (2) outputs an initial digital signal (S) of HDMI type, that is to say a high-definition digital audio-video signal of HDMI (High-Definition Multimedia Interface) type. Therefore The cable (20) connected to the camera must be an HDMI-type cable having an HDMI-type connector (21) that connects to an input port (30) of the video capture card (3).

The station (100) comprises a signal switch (4) and a PC (5) with a screen (55).

The video capture card (3) splits the initial signal (S) into a first signal (D) that is sent to the PC (5) and into a second signal (S2) that is sent to the signal switch (4). The first signal (D) and the second signal (S2) are a duplication of the initial signal (S).

The signal switch (4) comprises at least two input ports (41, 42), one output port (44), and a selector to select one or more input ports.

Advantageously, the signal switch (4) comprises three input ports:
- a first input port (41) identifying a first channel (CH1),
- a second input port (42) identifying a second channel (CH2), and
- a third input port (43) identifying a third channel (CH3).

The video capture card (3) is connected to the PC (5) via a USB 3.0 digital cable (50) having a first USB-A 3.0 type connector (51) connected to a second output port (32) of the video capture card (3) and a second USB-A 3.0 type connector (52) connected to a USB-A 3.0 type input port (53) of the PC (5).

In this way, the video capture card (3) sends the first signal (D) to the input port (53) of the PC (5) so that the screen (55) of the PC displays the images taken by the camera (2).

The PC (5) has an HDMI output port (54) for outputting a high-definition digital signal. If a screen duplication function is enabled in the PC (5), there will be a digital screen duplication signal (S1) of HDMI type in the HDMI output port (54) of the PC, which is a duplication of the video signal displayed in the screen (55) of the PC. Therefore, if an image taken by the camera (2) is displayed on the screen (55) of the PC, a screen duplication signal (S1) will be outputted from the HDMI output port (54) of the PC, which contains the image from the camera (2). The screen duplication signal (S1) is sent to the signal switch (4) via an HDMI cable (57) having a first connector (58) connected to the HDMI output port (54) of the PC and a second connector (59) connected to the first input port (41) of the analog signal switch.

The video capture card (3) is connected to the signal switch (4), via an HDMI cable (33) having a first connector (34) connected to a first output port (31) of the video capture card (3) and a second connector (35) connected to the second input port (42) of the signal switch (4).

The second signal (S2) from the video capture card (3) is sent to the second channel (CH2) of the second input port (42) of the signal switch.

The third input port (43) of the signal switch (4) is set for a possible external signal (S3) from external devices (400) to the multimedia dental station according to the invention.

Advantageously, the signal switch (4) may also comprise a signal splitter that splits a single or picture-to-picture HDMI signal coming from the three input signals (S1, S2, S3) into a first output signal (S4) and a second output signal (S5), which are always digital HDMI-type signals sent to the first output port (44) and to a second output port (45) of the signal switch, respectively.

The second output signal (S5) can be sent to an external device (600) provided with HDMI input port, such as a monitor, a video projector or a 5G wireless image transmitter for mobile devices.

The first output signal (S4) is sent to a transmitter (6) of wireless type responsible for transmitting the first output signal (S4). The first output port (44) of the signal switch (4) is directly coupled to an input port (60) of the transmitter (6).

The transmitter (6) is a 5.8 GHz HD 1080 p wireless type transmitter with 0.05 s latency.

The station (100) comprises a helmet assembly (G).

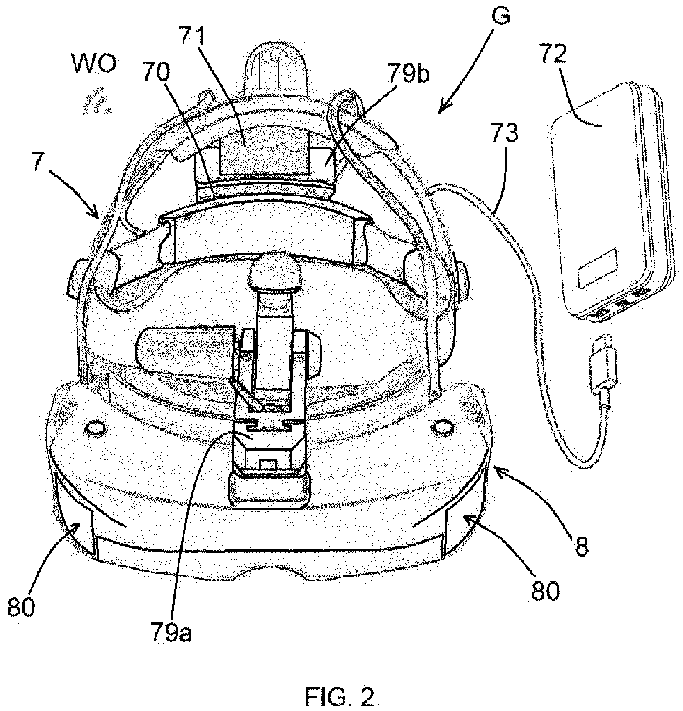

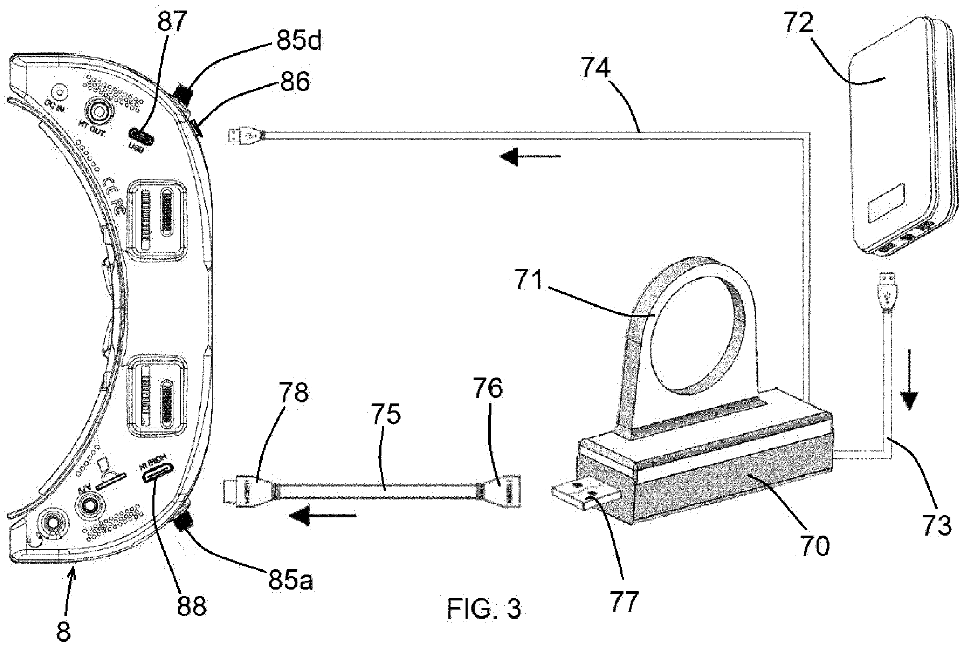

With reference to Figs. 2, 3 and 4, the helmet assembly (G) comprises:
- a dental prismatic helmet (7) suitable for being worn by the dentist,
- an OLED viewer (8) mounted on the prismatic helmet (7) in order to be viewed by the dentist,
- a receiver (70) mounted on the prismatic helmet, and
- a battery holder (71) mounted on the prismatic helmet.

The OLED viewer (8) comprises an organic LED display.

The receiver (70) of the helmet is wirelessly coupled (W0) (see Fig. 1) with the transmitter (6) to receive the first output signal (S4) from the signal switch (4).

The OLED viewer (8) and the receiver (70) are electrically powered by a power bank (72). The power bank (73) is connected to the receiver (70) via a first USB-C cable (73). A second USB-C cable (74) connects the receiver (70) to a USB port (87) of the OLED viewer (8) to send a 5 Vdc voltage to power the OLED viewer (8). The power bank (72) is magnetically attached to a dedicated holder.

The OLED viewer (8) is connected to the receiver (70) via a digital cable (75) of MINI HDMI type, having a first connector (76) connected to an HDMI output port (77) of the receiver (70) and a second connector (78) connected to a MINI HDMI input port (88) of the OLED viewer (8). In this way, the first output signal (S4) coming out of the signal switch (4) reaches the OLED viewer (8) and is displayed in the OLED viewer (8).

The OLED viewer (8) and the receiver (70) are both anchored on the prismatic helmet (7) by means of respective adapters (79a, 79b).

The OLED viewer (8) has at least one slot (80) suitable to accommodate a transmitter module.

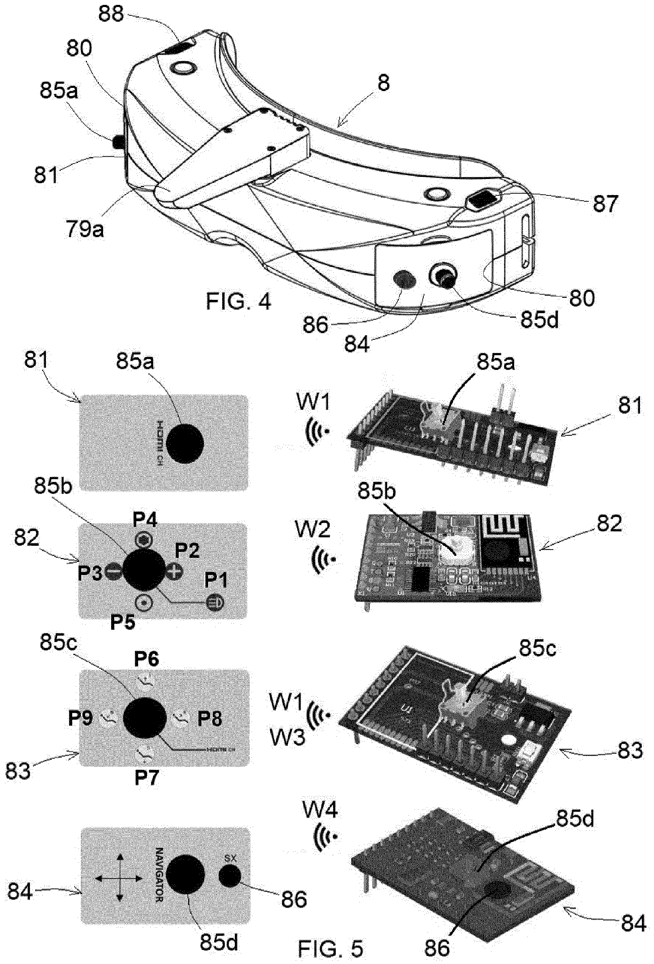

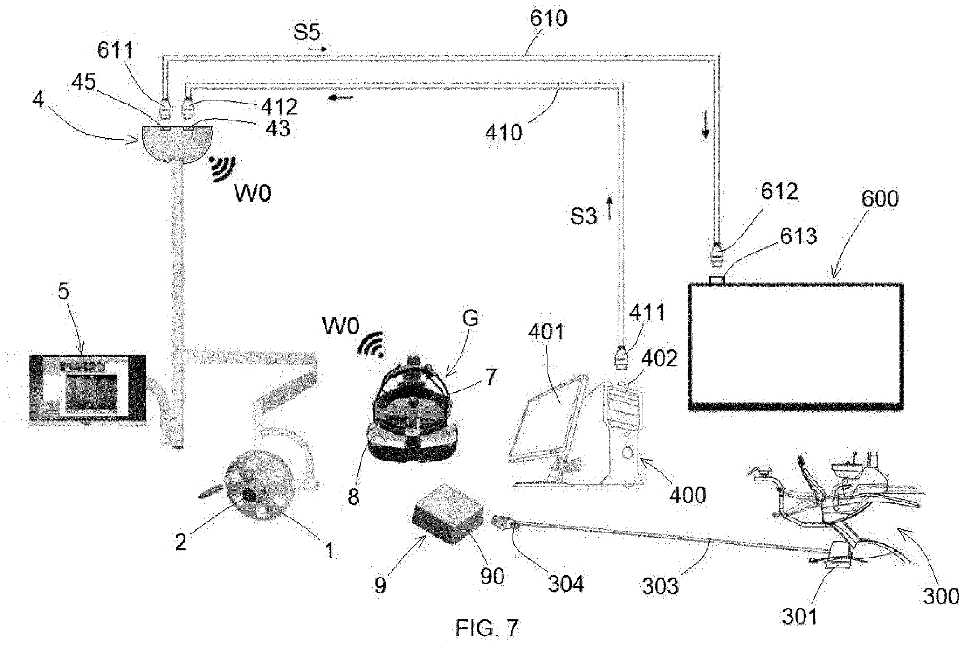

Referring to Fig. 5, the station (100) may comprise one or more of the following four transmitter modules:
- a switch transmitter module (81) to transmit commands to the signal switch (4),
- a lamp and camera transmitter module (82) to transmit commands to the lamp (1) and to the camera (2),
- a switch and patient support transmitter module (83) to transmit commands to the signal switch (4) and to a third receiver (9) suitable for being connected to a control unit (301) of a patient support (300) (see Fig. 7), and
- a PC transmitter module (84) for transmitting commands to the PC (5).

The functions implemented by the switch transmitter module (81) are minimum functions, whereas the functions implemented by the other three transmitter modules (82, 83, 84) are optional functions.

The switch transmitter module (81) has a transmitter that is wirelessly coupled with an integrated first receiver (46) of the signal switch (4) (Fig. 1), so as to control the selector of the signal switch (4) to select one of the three channels (CH1, CH2, CH3) of the signal switch. In this way, the dentist can select the reception of the desired analog signal on the OLED viewer (8) choosing between:
- the screen duplication signal (S1) entering the first channel (CH1),
- the second signal (S2) entering the second channel (CH2), and
- the external signal (S3) entering the third channel (CH3).

It should be considered that the screen duplication signal (S1) entering the first channel (CH1) can be the image taken by the camera (2) enriched with additional information available in the PC (5).

In this way, by selecting the second channel (CH2), the dentist will see the image taken by the camera (2) in the OLED viewer (8), whereas by selecting the first channel (CH1) the dentist will see the image taken by the camera enriched with additional information available in the PC (5) in the OLED viewer (8), without having to remove the prismatic helmet (7).

The dentist can also select a multi-image picture-to-picture function on the OLED viewer (8).

The switch transmitter module (81) is equipped with a joystick (85a) that can be operated by the dentist to choose the desired signal or select a multi-image, which simultaneously shows 2/3 input signals. The joystick (85a) can be a four-axis analog micro-joystick with a central SW button.

By repeatedly pressing the joystick (85a), the dentist can control all the parameters of the signal switch (4), such as:
- selection of the first channel (CH1),
- selection of the second channel (CH2),
- selection of the third channel (CH3),
- selection of a multi-image obtained from the images of the first channel (CH1) and of the second channel (CH2),
- selection of a multi-image obtained from the images of all three channels (CH1, CH2, CH3).

The selected type of signal will be split and present in the two outputs (44, 45) of the signal switch (4) in order to be sent also to an external device.

The lamp and camera transmitter module (82) has a transmitter that is wirelessly coupled (W2) to a second receiver (23) integrated into the operating lamp (1) and connected to the camera (2) to control the main operating parameters of the lamp (1) and of the camera. The lamp and camera transmitter module (81) is equipped with a joystick (85b), which can be a four-axis analog micro-joystick with a central SW button. By using the joystick (85b), the dentist can control the operating parameters of the lamp and camera, such as:
- turning on, turning off and adjustment of the lamp brightness (P1)
- zoom + (P2);
- zoom - (P3);
- diaphragm opening (P4); and
- diaphragm closing (P5).

The switch and patient support transmitter module (83) has a transmitter that is wirelessly coupled (W1) to the first receiver (46) integrated into the signal switch (4) and wirelessly coupled (W3) to the third receiver (9) suitable for being connected to the control unit (301) of the patient support (300). The patient support (300) is of motorized type with electric motors suitable for changing the position of the patient support (300). The electric motors are driven by the control unit (301). The patient support (300) can be a dental chair or an operating table.

The switch and patient support transmitter module (83) enables all functions of the switch transmitter module (81) and also functions related to the control of the patient support. Therefore, it can be used in place of the switch transmitter module (81).

The switch and patient support transmitter module (83) is equipped with a joystick (85c), which can be a four-axis analog micro-joystick with a central SW button.

The joystick (85c) of the switch and patient support transmitter module (83) can be operated by the dentist to perform all the functions described with reference to the switch transmitter module (81).

In addition, with the joystick (85c) of the switch and patient support transmitter module (83) the dentist can control the opening or closing of four static relays provided in the third receiver (9) suitable for being connected to the control unit (300) of the patient support (300), which control four motors provided in the patient support (300).

Given that the patient support (300) has a movable seat and a movable backrest actuated by means of electric motors, by means of the axial movements of the joystick (85c), the dentist can control the movements of the patient support (300), such as:
- seat raising (P6),
- seat descent (P7),
- backrest raising (P8),
- backrest descent (P9),

The PC transmitter module (84) has a transmitter that is wirelessly coupled (W4) to a fourth receiver (56) having a USB-A type output connected to the PC (5) to manage the functions of the PC (5). The PC transmitter module (84) has an analog joystick (85d) with potentiometers, a central SW button and a tactile button (86) next to the joystick (85d).

By using the joystick (85d) and the tactile button (86) the dentist can replicate all the functions of an ordinary mouse connected to the PC (5);
- the axial movements of the joystick (85d) enable a multi-directional movement of the cursor on the screen (55) of the PC;
- a compression of the joystick (85d) simulates a press of the mouse right button;
- a compression of the tactile button (86) simulates a press of the mouse left button.

All the transmitter modules (81, 82, 83, 84) located in the housings (80) of the OLED viewer (8) transmit control signals in a frequency range of 2.4 GHz.

Referring to Fig. 4, the OLED viewer (33) by means of an adapter (53), and the wireless analog receiver (40) are anchored on the prismatic helmet (32).

The OLED viewer (8) has the switch transmitter module (81) or the switch and patient support transmitter module (83). If the dentist selects the first channel (CH1) of the signal switch (4), the OLED viewer (8) displays a duplication of the images displayed on the screen (55) of the PC. The use of the first channel (CH1) can be useful when the dentist needs to consult a patient file stored in the PC (5), or when the dentist wants to view a live capture of the patient's oral cavity with an intraoral camera, or when the dentist wants to view a live capture of a 3D digital dental impression. In fact, the capture of the 3D digital dental impression can only be seen on the screen (55) of the PC with a powerful graphics card.

If the dentist selects the second channel (CH2), the OLED viewer (33) displays the shooting of the camera (2) live.

If, on the other hand, the dentist selects the third channel (CH3), the OLED viewer (8) displays a duplication of the images displayed on an external PC, such as the PC dedicated to radiological imaging.

If, during the surgical intervention, the dentist selects the multi-image function, the OLED viewer (8) will display:
- a live shooting of the camera in 4:3 format coming from the second channel (CH2),
- a radiological image of the patient's oral cavity in reduced 4:3 format coming from the third channel (CH3), and
- any information reproduced on the screen (55) of the PC (5) in reduced 4:3 format coming from the first channel CH1.

By using the joystick (38b), the lamp and camera transmitter module (82) makes it possible to easily and quickly change the main operating parameters of the camera (2) during the procedure, such as switch on and off, brightness adjustment (P1), zoom + (P2); zoom - (P3); diaphragm aperture (P4) and diaphragm closing (P5).

During the configuration of the station (100), the dentist can choose to configure the OLED viewer (8) with different transmitter modules according to the specific needs, such as:
- the PC transmitter module (84) to replicate the functions of a mouse via the joystick (85d) and the tactile button (86), so as to manage the PC (5) by plugging the fourth receiver (56) into a USB port of the PC,
- the switch and patient support transmitter module (83), which, in addition to having the functions of the switch transmitter module (81), makes it possible to easily and quickly adjust the patient's position during surgery with the joystick (38c). The axial movements of the joystick (38c) operate the motors for seat rise (P6), seat descent (P7), backrest rise (P8) and backrest descent (P9) on a dental chair or an operating table.

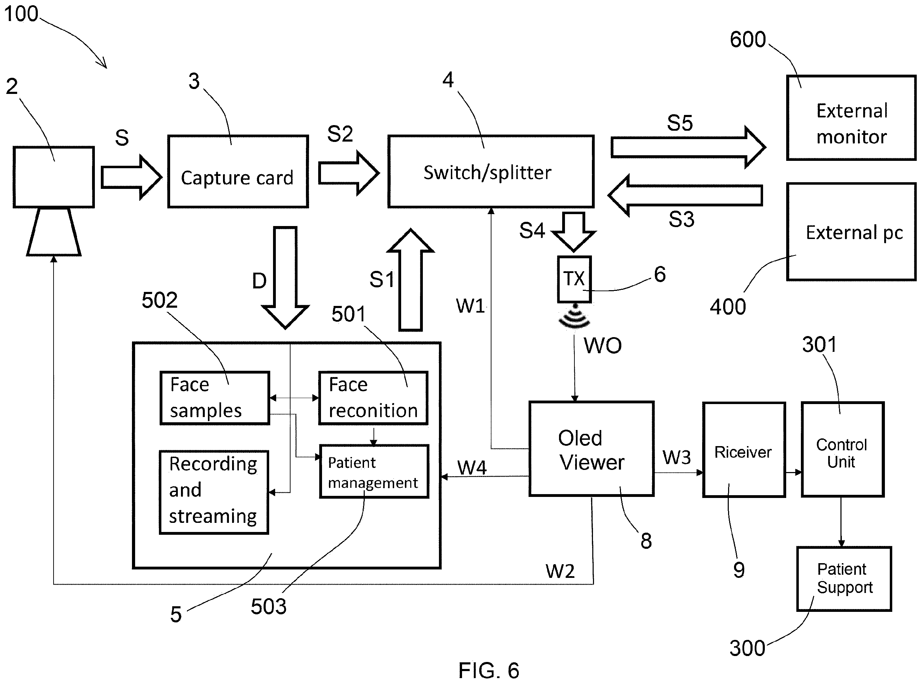

With reference to Fig. 6, the PC (5) has a face recognition software (501), such as for instance an open-source software (OpenFace/OpenCV). Such face recognition software (501) can recognize a face taken by the camera (2) or by an intraoral camera or by a 3D intraoral scanner connected to the PC (5), comparing the face with sample faces (502) stored in the PC.

The PC has a patient management program (503) in which the data and the medical files of each patient are stored. In such a case, the data stored for each patient will include the patient's face captured by the camera (2) and sent to the PC (5). In this way, a library of sample faces (502) is created in the PC (5), which are used for a comparison performed by the face recognition software (501). Each sample face (502) is linked to the medical file of a respective client contained in the patient management program (503).

Therefore, when a patient is to undergo a dental session, the camera (2) will frame the patient's face, and the face recognition software (501) will recognize the patient's face among the various sample faces (502) and will open the patient's medical file contained in the patient management program (503) that will be displayed on the screen (55) of the PC.

In such a case, without having to remove the prismatic helmet (7), by selecting the first channel (CH1), the dentist will display the patient file on the OLED viewer (8), and the patient file will be displayed on the screen (55) of the PC.

As shown in Fig. 7, the station (100) can be connected to external devices, such as the patient support device (300), an external computer (400) with a screen (401), an external monitor (600), such as a monitor installed on a wall.

Specifically, the station (100) enables:
- to display the screen (401) of the external PC (400) on the OLED viewer (8),
- to display what the dentist sees in the OLED viewer (8) on the external monitor (600),
- to control the movements of the patient support (300) with the joystick (85c) of the switch and patient support transmitter module (83).

To display the screen (401) of the external PC (400) on the OLED viewer (8), it is necessary to activate the screen duplication function on the external PC (400).

The external PC (400) has an HDMI output port (402). If the screen duplication function is enabled in the external PC (400), a screen duplication signal (S3) of HDMI type, which is a duplication of the video signal displayed on the screen (401) of the external PC (400), will be present from the HDMI output port (402) of the external PC. The screen duplication signal (S3) is sent to the signal switch (4) by means of an HDMI cable (410) having a first connector (411) connected to the HDMI output port (402) of the external PC and a second connector (412) connected to the third input port (43) of the signal switch (4) that identifies the third channel (CH3).

To display what the dentist sees in the OLED viewer (8) on the external monitor (600), the second output signal (S5) coming out of the signal switch (4) must be sent to the external monitor (600). In fact, the second output signal (S5) is a duplicate of the first output signal (S4) that is sent to the OLED viewer (8). The external monitor (600) is connected to the signal switch (4) via an HDMI cable (610) having a first HDMI connector (611) connected to the second output port (45) of the signal switch (4) and a second HDMI connector (612) connected to an HDMI input port (613) of the external monitor (600).

To control the movements of the patient support (300) with the joystick (85c) of the switch and patient support transmitter module (83), the third receiver (9) of the patient support (300) must be connected to the control unit (301) that controls the motors of the patient support.

The movements of the patient support are controlled by the joystick (85c) of the switch and patient support transmitter module (83). The third receiver (9) is connected to the control unit (301) of the patient support by a cable (303) equipped with a connector (304) that is engaged in a port (90) of the third receiver (9).

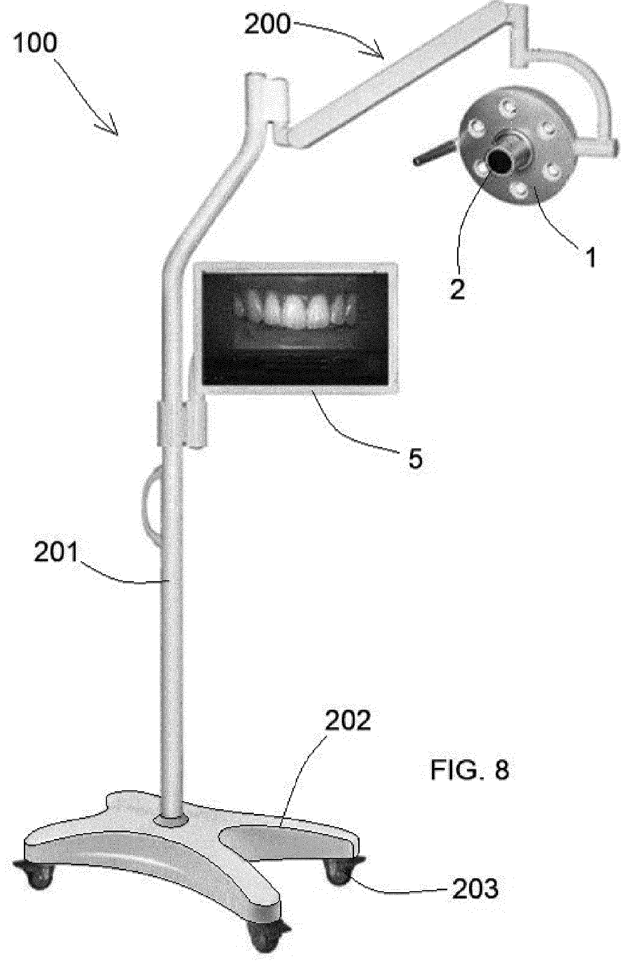

Fig. 8 illustrates a first type of installation of the multimedia dental station according to the invention, of wheeled stand type, in which the articulated system (200) supporting the lamp (1) is mounted on an upright (201) supported by feet (202) mounted on swivel wheels (203). The PC (5) is supported by the upright (201).

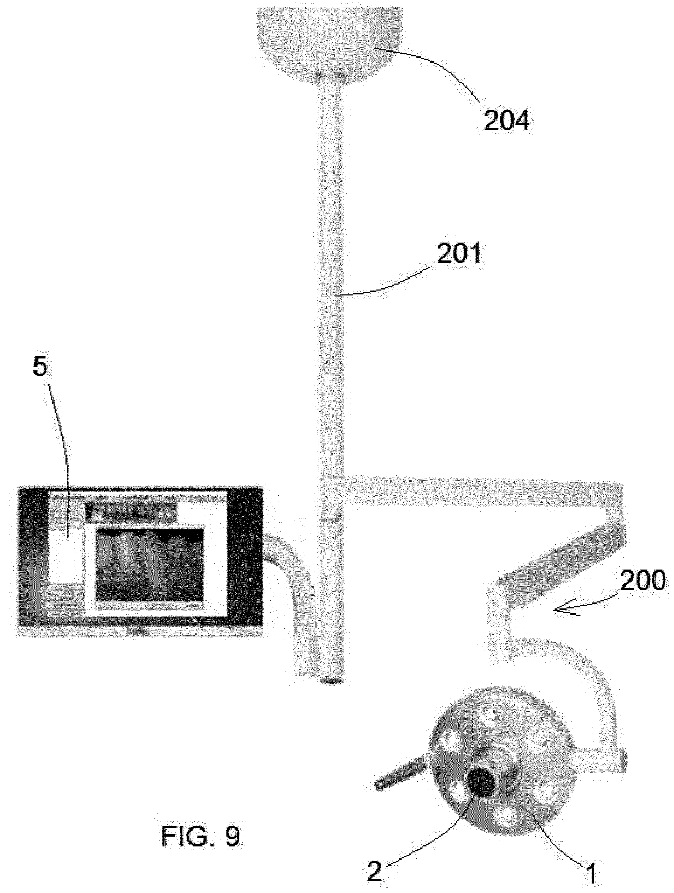

Fig. 9 illustrates a second type of installation of the multimedia dental station according to the invention, of ceiling-mounted type, in which the articulated system (200) supporting the lamp (1) is mounted on an upright (201) fixed to a ceiling by means of an attachment (204). The PC (5) is supported by the upright (201).

The station (100) in the two variants described above is capable of performing the following functions:
- to perfectly illuminate the operating field by means of the lamp (1);
- to shoot the surgical intervention by means of the camera (2),
- to share images and videos of the surgical intervention with the patient on the screen (55) of the PC (5) or on the external monitor (600) tuned to the first channel (CH1),
- to share the video of the surgical intervention in streaming via the PC (5) that receives the video signal from the camera (2),
- to view the shooting of the operating field in real time on the screen (55) of the PC (5) with up to 30x magnification,
- to view the operating field at 30x magnification by means of the camera (2) on the OLED viewer (8) anchored to the prismatic helmet (7) of the dentist by selecting the second channel (CH2),
- to view a duplication of the screen (55) of the PC (5) on the OLED viewer (8), selecting the first channel (CH1), thus facilitating an acquisition of the 3D digital dental impression;
- to view a duplication of the screen (401) of the external PC (400) on the OLED viewer (8), selecting the third channel (CH3), making available relevant information necessary for the success of the intervention;
- to simultaneously view three video sources transmitted on the three channels (CH1, CH2, CH3) on the OLED viewer (8), selecting the multi-image mode, so that the dentist can operate in real work with zoom up to 30X, having all the relevant clinical information necessary for the success of the intervention;
- to work without having to wear glasses thanks to a dioptric adjustment of the OLED viewer (8);
- to adjust the parameters (P1, P2, P3, P4, P5) of the lamp (1) and camera (2) directly with the joystick (85b) of the OLED viewer (8), without taking the eyes off the operating field;
- to adjust the position of the patient support (300) directly with the joystick (85c) of the OLED viewer (8), without looking away from the operating field;
- to access all functions of the PC (5) or of the external PC (400) directly with the joystick (85d) and the tactile button (86) of the OLED viewer (8), to search and consult the clinical information necessary for the success of the surgery, without taking the eyes off the operating field;
- to open the patient file thanks to the face recognition software (501), as soon as the patient sits in the dental chair and is framed by the camera (2);
- to store the audio-video-photo documents taken by the camera (2) in the patient file of the patient management program (503).

All of these functions of the station (100) allow the dentist to increase his/her concentration, isolating him/her from external visual stimuli and improving his/her operating posture. With the streaming or sharing function in remote mode, the station (100) can meet the needs of telemedicine, in those cases in which a doctor or medical team offers remote support to another doctor or medical team, either in cases of emergency or in cases where it is impossible to go directly to the site. In such a case, the digital twin technique can be used to enable a visual support for the medical team that is performing the intervention.

Numerous variations and detailed modifications can be made to the present embodiment of the invention, within the reach of a person skilled in the art, while still falling within the scope of the invention as expressed by the appended claims.

## Claims

1. Multimedia dental station (100) comprising:
- an operating lamp (1) suitable for illuminating the patient's oral cavity,
- a video camera (2) integrated in the operating lamp (1) for taking images and films of the patient's oral cavity,
- a signal switch (4) having a selector for selecting at least a first channel (CH1) and a second channel (CH2)
- a transmitter (6) connected to the signal switch (6) for transmitting a first output signal (S4),
- a device (5) having a screen (55) suitable for displaying the images taken by the video camera (2),
- a prismatic helmet (7) suitable for being worn by the dentist,
- a receiver (70) mounted on the prismatic helmet (7) to receive said first output signal (S4) from the transmitter (6),
- a viewer (8) mounted on the prismatic helmet (32) and connected to said receiver (70) to display said first output signal (S4) from said transmitter (6), and
- a switch transmitter module (81) mounted in said viewer (8) and coupled to a first receiver (46) integrated in said signal switch (4) to send command signals to control said selector of the signal switch in such a way to select one or more of said channels (CH1, CH2) of said signal switch,
**characterized in that**
said viewer (8) is an OLED viewer;
said station (100) comprises a video capture card (3) connected to the camera (2) and configured to split an initial signal (S) coming from the camera into a first signal (D) sent to said device (5) and a second signal (S2) sent to said signal switch (4),
said signal switch (4) has a first input port (41) identifying said first channel (CH1), a second input port (42) identifying said second channel (CH2) and a first output port (44) and a selector to select one or more input ports;
said video capture card (3) is connected to the second input port (42) of the second channel (CH2) of the signal switch (4) to send said second signal (S2) to the signal switch (4),
said transmitter (6) is of wireless type and is connected to the first output port (44) of the signal switch (40) to receive the first output signal (S4), according to the selection of one or more input ports of the signal switch,
said device (5) is a personal computer (PC) connected to the video capture card (3) to receive said first signal (D) from the video capture card (3) and displaying the images taken by the camera (2) on the screen (55) of the PC;
said PC (5) has an output port (54) for outputting a screen duplication signal (S1) which is a duplication of the video signal of the images displayed on the screen (55) of the PC, wherein said output port (54) of the PC (5) is connected to the first input port (41) of the first channel (CH1) of the signal switch (4) to send said screen duplication signal (S1) to the signal switch (4), so that by selecting the second channel (CH2) the dentist sees the image taken by the camera (2) in the OLED viewer (8), whereas by selecting the first channel (CH1), the dentist sees the image taken by the camera (2) enriched with additional information available on the PC in the OLED viewer (8).

2. The station (100) according to claim 1, wherein said switch transmitter module (81) comprises a joystick (85a) that is used by the dentist to select one or more of said channels (CH1, CH2) of said signal switch.

3. The station (100) according to claim 1 or 2, comprising a lamp and camera transmitter module (82) mounted in said OLED viewer (8) and wirelessly coupled (W2) to a second receiver (23) of the operating lamp (1) connected to the camera (2) to control the operating parameters of the lamp (1) and of the camera (2); wherein said lamp and camera transmitter module (82) comprises a joystick (85b) that is used by the dentist to control the lamp and the camera.

4. The station (100) according to any one of the preceding claims, comprising:
- a third receiver (9) suitable for being connected to a control unit (301) of a user support device (300), such as a dental chair or an operating table of motorized type, wherein electric motors adjust the position of the user support device (300), and
- a switch and patient support transmitter module (83) mounted in said OLED viewer (8) and wirelessly coupled (W3) to said third receiver (9) to control said electric motors of the patient support; wherein said switch and patient support transmitter module (83) is provided with a joystick (85c) that is used by the dentist to control said electric motors of said patient support.

5. The station (100) according to any one of the preceding claims, comprising:
- a fourth receiver (56) connected to said PC (5), and
- a PC transmitter module (84) mounted in said OLED viewer (8) and wirelessly coupled (W4) to said fourth receiver (56) to replicate the functions of a mouse in said PC; wherein said PC transmitter module (84) is provided with a joystick (85d) and a touch button (86) that are used by the dentist to replicate the functions of a mouse in said PC.

6. The station (100) according to any one of the preceding claims, wherein said signal switch (4) comprises a third input port (43) identifying a third channel (CH3) suitable for being connected to an external PC (400).

7. The station (100) according to claim 6, wherein said external PC (400) has a screen (401) and an HDMI output port (402) which outputs a screen duplication signal (S3) of HDMI type, which is a duplication of the video signal of the images displayed on the screen (401) of the external PC; wherein said HDMI output port (402) of the external PC is connected to said third input port (43) of the third channel of the signal switch.

8. The station (100) according to any one of the preceding claims, wherein said signal switch (4) has a second output port (45) and is also a signal splitter that splits an HDMI signal coming from one or more of the input ports (41, 42, 43) into said first output signal (S4) and into a second output signal (S5) of HDM type, respectively sent on the first output port (44) and on the second output port (45) of the signal switch; wherein the second output signal (S4) is identical to the first output signal (S3).

9. The station (100) according to any one of the preceding claims, wherein said camera (1) has an optical zoom of at least 30 X.

10. The station (100) according to any one of the preceding claims, wherein said transmitter (6) is a 5.8 GHz transmitter that is set to transmit the first output signal (S4) on the receiver (70) disposed on the prismatic helmet; wherein said receiver (70) is a 5.8 GHz receiver connected to an HDMI input port (88) of the OLED viewer (8).

11. The station (100) according to any one of the preceding claims, wherein said PC (5) comprises:
- a face recognition software (501) capable of recognizing a face taken by the camera (2) and comparing said face with sample faces (502) stored in the PC (5), and
- a patient management program (503) wherein the data and the medical files of each patient are stored; wherein each sample face (502) stored in the PC (5) is linked to the medical file of a respective patient, and
wherein the PC (5) is configured to display the medical file of a patient on the screen (55) when the face recognition software (501) recognizes a face of a patient taken by the camera (2).

12. The station (100) according to any one of the preceding claims, wherein said operating lamp (1) is mounted on an articulated system (200) supported by an upright (201) that supports said PC (5); wherein said upright (201) is mounted on feet (202) mounted on swiveling wheels (203) or is fixed to a ceiling by means of an attachment (204).
